(19) Europäisches Patentamt
European Patent Office
Office européen des brevets

(11) **EP 4 403 094 A1**

(12) **EUROPEAN PATENT APPLICATION**
published in accordance with Art. 153(4) EPC

(43) Date of publication:
**24.07.2024  Bulletin 2024/30**

(21) Application number: **22870286.6**

(22) Date of filing: **14.09.2022**

(51) International Patent Classification (IPC):
*A61B 5/00* (2006.01)     *A61B 5/024* (2006.01)
*A61B 5/021* (2006.01)     *A61B 5/1455* (2006.01)
*G16H 50/20* (2018.01)

(52) Cooperative Patent Classification (CPC):
**A61B 5/00; A61B 5/021; A61B 5/024;
A61B 5/1455; G16H 50/20; G16H 50/30**

(86) International application number:
**PCT/KR2022/013741**

(87) International publication number:
**WO 2023/043202 (23.03.2023 Gazette 2023/12)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.09.2021  KR 20210124276
16.09.2021  KR 20220042151**

(71) Applicant: **Sky Labs Inc.
Seongnam-si, Gyeonggi-do 13486 (KR)**

(72) Inventors:
• **CHANG, Hyung Min**
**Yongin-si, Gyeonggi-do 16843 (KR)**
• **SEOL, Ki Hyuk**
**Seoul 05616 (KR)**
• **KIM, Chang Hyun**
**Seoul 05372 (KR)**
• **CHOI, Chang Woo**
**Uiwang-si, Gyeonggi-do 16000 (KR)**
• **LEE, Byung Hwan**
**Yongin-si, Gyeonggi-do 16981 (KR)**

(74) Representative: **Loyer & Abello
9, rue Anatole de la Forge
75017 Paris (FR)**

(54) **BIOMETRIC DATA MONITORING PLATFORM USING BIOMETRIC SIGNAL SENSING RING**

(57)     Provided is a biometric data monitoring platform using a biometric signal sensing ring. The platform includes a server, wherein the server includes a signal quality classification system including a first signal quality classification component configured to classify signal quality of a first wavelength photoplethysmography (PPG) signal as being good or bad, a biometric data calculation system including an atrial fibrillation determination component configured to determine whether atrial fibrillation has occurred, from the first wavelength PPG signal by using a deep learning model, and a biometric index calculation system including an atrial fibrillation index calculation component configured to calculate an atrial fibrillation index from a first signal quality classification result generated by the first signal quality classification component and an atrial fibrillation determination result generated by the atrial fibrillation determination component.

FIG. 3A

## Description

Technical Field

The present disclosure relates to a biometric data monitoring platform. More particularly, the present disclosure relates to a biometric data monitoring platform using a biometric signal sensing ring.

Background Art

Continuous monitoring of a biometric signal and biometric data obtained by processing the biometric signal is useful for prevention, an early diagnosis, and management of disease. To develop such a biometric data monitoring platform, a biometric signal sensing apparatus for easily and continuously measuring a biometric signal in everyday life with high reliability is required.

Disclosure

Technical Problem

Provided is a biometric data monitoring platform using a biometric signal sensing ring.

Technical Solution

According to an embodiment of the present disclosure, a biometric data monitoring platform using a biometric signal sensing ring, includes a server, wherein the server includes a signal quality classification system including a first signal quality classification component configured to classify signal quality of a first wavelength photoplethysmography (PPG) signal as being good or bad, a biometric data calculation system including an atrial fibrillation determination component configured to determine whether atrial fibrillation has occurred, from the first wavelength PPG signal by using a deep learning model, and a biometric index calculation system including an atrial fibrillation index calculation component configured to calculate an atrial fibrillation index from a first signal quality classification result generated by the first signal quality classification component and an atrial fibrillation determination result generated by the atrial fibrillation determination component, wherein the atrial fibrillation index is defined by a ratio of a time when quality of the first wavelength PPG signal is classified as being good by the first signal quality classification component to a time when the quality of the first wavelength PPG signal is classified as being good by the first signal quality classification component and the atrial fibrillation is determined to have occurred by the atrial fibrillation determination component.

According to some embodiments, the signal quality classification system may further include a second signal quality classification component configured to classify signal quality of a second wavelength PPG signal as being good or bad, and the biometric data calculation system may further include a signal feature extraction component configured to extract, from the second wavelength PPG signal, a PPG feature by using a first deep learning model, a user feature extraction component configured to extract a user feature by using a second deep learning model, from user information, a test PPG signal measured by using the biometric signal sensing ring, and systolic and diastolic test blood pressure measured simultaneously with the test PPG signal by using a general blood pressure gauge, and a blood pressure estimation component configured to estimate systolic and diastolic blood pressure from the PPG feature and the user feature by using a third deep learning model.

According to some embodiments, the biometric index calculation system may further include a blood pressure index calculation component configured to calculate a blood pressure index from blood pressure estimated by the blood pressure estimation component and a second signal quality classification result generated by the second signal quality classification component, and the blood pressure index may be defined by a ratio of a time when quality of the second wavelength PPG signal is classified as being good by the second signal quality classification component to a time when the quality of the second wavelength PPG signal is classified as being good by the second signal quality classification component and the systolic blood pressure is outside a first normal range or the diastolic blood pressure is outside a second normal range.

According to some embodiments, the signal quality classification system may further include a third signal quality classification component configured to classify quality of a second wavelength PPG signal and a third wavelength PPG signal as being good or bad, and the biometric data calculation system may further include an oxygen saturation estimation component configured to estimate oxygen saturation from the second wavelength PPG signal and the third wavelength PPG signal.

According to some embodiments, the biometric index calculation system may further include an oxygen saturation index calculation component configured to calculate an oxygen saturation index from oxygen saturation estimated by the oxygen saturation estimation component and a third signal quality classification result generated by the third signal quality classification component, and the oxygen saturation index may be defined by a ratio of a time when the quality of the second wavelength PPG signal and the third wavelength PPG signal is classified as being good by the third signal quality classification component to a time when the quality of the second wavelength PPG signal and the third wavelength PPG signal are classified as being good by the third signal quality classification component and the oxygen saturation is outside a normal range.

According to some embodiments, the first wavelength PPG signal, the second wavelength PPG signal, and the third wavelength PPG signal may be measured by using the biometric signal sensing ring and the server receives the first wavelength PPG signal, the second wavelength PPG signal, and the third wavelength PPG signal from the biometric signal sensing ring through a terminal, the biometric signal sensing ring may include a plurality of sensors configured to simultaneously measure a plurality of first wavelength PPG signals, a plurality of second wavelength PPG signals, and a plurality of third wavelength PPG signals at different locations, respectively, and each of the plurality of sensors may include a first wavelength light source, a second wavelength light source, a third wavelength light source, and a photoelectric conversion device.

According to some embodiments, the terminal may include a light source control component configured to control the first wavelength light source, the second wavelength light source, and the third wavelength light source of each of the plurality of sensors such that a direct current (DC) component of each of first wavelength test PPG signals measured by using the plurality of sensors is within a first predetermined range, a DC component of each of a plurality of second wavelength test PPG signals measured by using the plurality of sensors is within a second predetermined range, and a DC component of each of a plurality of third wavelength test PPG signals measured by using the plurality of sensors is within a third predetermined range.

According to some embodiments, the terminal may further include a sensor selection component configured to select, from among the plurality of sensors, as a sensor for measuring the first wavelength PPG signal, the second wavelength PPG signal, and the third wavelength PPG signal, a sensor that measures a combination of a first wavelength test PPG signal, a second wavelength test PPG signal, and a third wavelength test PPG signal, which has highest signal quality, from among the plurality of first wavelength test PPG signals, the plurality of second wavelength test PPG signals, and the plurality of third wavelength test PPG signals.

According to some embodiments, signal quality of the plurality of first wavelength test PPG signals, the plurality of second wavelength test PPG signals, and the plurality of third wavelength test PPG signals may be evaluated by at least one of magnitude of an acceleration signal, a signal-to-noise ratio, and a ratio of a DC component size to an alternating current (AC) component size.

According to some embodiments, the controlling of the first wavelength light source, the second wavelength light source, and the third wavelength light source of each of the plurality of sensors by the light source control component and the selecting of the sensor by the sensor selection component may be sequentially performed, and the controlling of the first wavelength light source, the second wavelength light source, and the third wavelength light source of each of the plurality of sensors by the light source control component and the selecting of the sensor by the sensor selection component may be periodically performed.

According to an embodiment of the present disclosure, a biometric data monitoring platform using a biometric signal sensing ring, includes a terminal, wherein the terminal is configured to receive a photoplethysmography (PPG) signal from a biometric signal sensing ring configured to measure the PPG signal, and transmit the PPG signal to a server, the biometric signal sensing ring includes a plurality of sensors configured to simultaneously measure a plurality of PPG signals at different locations, respectively, and each of the plurality of sensors includes a light source and a photoelectric conversion device.

According to some embodiments, the terminal may include a light source control component configured to control the light source of each of the plurality of sensors such that a direct current (DC) component of each of a plurality of test PPG signals measured by using the plurality of sensors is within a predetermined range.

According to some embodiments, the terminal may include a sensor selection component configured to select, from among the plurality of sensors, as a sensor for measuring the PPG signal, a sensor that measures a test PPG signal with highest signal quality.

According to some embodiments, signal quality of the plurality of test PPG signals may be evaluated by at least one of magnitude of an acceleration signal, a signal-to-noise ratio, and a ratio of a DC component size to an alternating current (AC) component size.

According to some embodiments, the controlling of the light source of each of the plurality of sensors by the light source control component and the selecting of the sensor by the sensor selection component may be sequentially performed, and the controlling of the light source of each of the plurality of sensors by the light source control component and the selecting of the sensor by the sensor selection component may be periodically performed.

According to an embodiment of the present disclosure, a biometric data monitoring platform using a biometric signal sensing ring, includes a terminal, wherein the terminal is configured to receive a first wavelength PPG signal, a second wavelength PPG signal, and a third wavelength PPG signal from the biometric signal sensing ring configured to measure the first wavelength PPG signal, the second wavelength PPG signal, and the third wavelength PPG signal, and transmit the first wavelength PPG signal, the second wavelength PPG signal, and the third wavelength PPG signal to a server, the biometric signal sensing ring includes a plurality of sensors configured to simultaneously measure a plurality of first wavelength PPG signals, a plurality of second wavelength PPG signals, and a plurality of third wavelength PPG signals at different locations, respectively, and each of the plurality of sensors includes a first wavelength light source, a second wavelength light source, a third wavelength light source, and a photoelectric conversion device.

According to some embodiments, the terminal may include a light source control component configured to control the first wavelength light source, the second wavelength light source, and the third wavelength light source of each of the plurality of sensors such that a direct current (DC) component of each of first wavelength test PPG signals measured by using the plurality of sensors is within a first predetermined range, a DC component of each of a plurality of second wavelength test PPG signals measured by using the plurality of sensors is within a second predetermined range, and a DC component of each of a plurality of third wavelength test PPG signals measured by using the plurality of sensors is within a third predetermined range.

According to some embodiments, the terminal may further include a sensor selection component configured to select, from among the plurality of sensors, as a sensor for measuring the first wavelength PPG signal, the second wavelength PPG signal, and the third wavelength PPG signal, a sensor that measures a combination of a first wavelength test PPG signal, a second wavelength test PPG signal, and a third wavelength test PPG signal, which has highest signal quality, from among the plurality of first wavelength test PPG signals, the plurality of second wavelength test PPG signals, and the plurality of third wavelength test PPG signals.

According to some embodiments, signal quality of the plurality of first wavelength test PPG signals, the plurality of second wavelength test PPG signals, and the plurality of third wavelength test PPG signals may be evaluated by at least one of magnitude of an acceleration signal, a signal-to-noise ratio, and a ratio of a DC component size to an alternating current (AC) component size.

According to some embodiments, the controlling of the first wavelength light source, the second wavelength light source, and the third wavelength light source of each of the plurality of sensors by the light source control component and the selecting of the sensor by the sensor selection component may be sequentially performed, and the controlling of the first wavelength light source, the second wavelength light source, and the third wavelength light source of each of the plurality of sensors by the light source control component and the selecting of the sensor by the sensor selection component may be periodically performed.

Advantageous Effects

Provided is a biometric data monitoring platform using a biometric signal sensing ring, which easily and continuously measures a biometric signal in everyday life with high reliability.

Description of Drawings

FIG. 1 is a 3-dimensional view of a biometric signal sensing ring according to an embodiment of the present disclosure.
FIG. 2 is an exploded 3-dimensional view of a biometric signal sensing ring according to an embodiment of the present disclosure.
FIG. 3A is a block diagram of a biometric data monitoring platform using a biometric signal sensing ring, according to an embodiment of the present disclosure.
FIG. 3B is a block diagram of a server according to an embodiment of the present disclosure.
FIGS. 4A and 4B are graphs showing examples of first wavelength photoplethysmography (PPG) signals before preprocessing.
FIGS. 4C and 4D are graphs showing examples of the first wavelength PPG signals of FIGS. 4A and 4B after preprocessing, respectively.
FIGS. 5A and 5B are graphs showing examples of first wavelength PPG signals classified as having good quality.
FIGS. 5C and 5D are graphs showing examples of first wavelength PPG signals classified as having bad quality.
FIGS. 6A and 6B are graphs showing examples of first wavelength PPG signals not determined as atrial fibrillation.
FIGS. 6C and 6D are graphs showing examples of first wavelength PPG signals determined as atrial fibrillation.
FIG. 7 is a block diagram of a signal feature extraction component, a user feature extraction component, and a blood pressure estimation component.

Mode for Invention

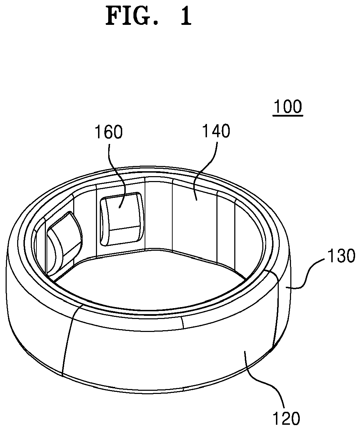

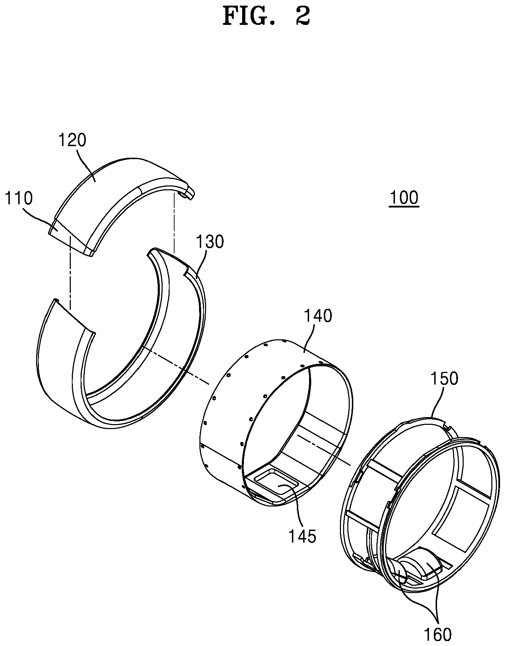

FIG. 1 is a 3-dimensional view of a biometric signal sensing ring 100 according to an embodiment of the present disclosure. FIG. 2 is an exploded 3-dimensional view of the biometric signal sensing ring 100 according to an embodiment of the present disclosure.

Referring to FIGS. 1 and 2, the biometric signal sensing ring 100 may include an outer electrode 130, an inner electrode 140, an insulating unit 150, a top cover 110, an operation display unit 120, and a plurality of sensors 160.

The outer electrode 130 may have an arch shape. The outer electrode 130 may include a conductor and function as an electrode for measuring electrocardiogram (ECG). Also, the outer electrode 130 may form the outer appearance of the biometric signal sensing ring 100 and be in contact with the body of a user.

The inner electrode 140 may have a ring shape and include a plurality of openings 145 for the plurality of sensors 160. The inner electrode 140 may include a conductor and function as an electrode for measuring ECG. Also, the inner electrode 140 may form the inner appearance of the biometric signal sensing ring 100 and be in contact with a finger of the user.

The insulating unit 150 may be provided between the outer electrode 130 and the inner electrode 140. The insulating unit 150 may enable electric insulation between the outer electrode 130 and the inner electrode 140.

The top cover 110 may have an arc shape and form a ring shape together with the outer electrode 130. The top cover 110 may form the outer appearance of the biometric signal sensing ring 100.

The operation display unit 120 may be combined with the top cover 110. The operation display unit 120 may include a plurality of light-emitting diodes (LEDs), for example, a green LED and a red LED. The operation display unit 120 may display an operation of the biometric signal sensing ring 100 by using the plurality of LEDs. For example, the green LED may be turned on for 2 seconds to indicate the start of measurement, and the green LED may be turned on for 1 second to indicate the end of the measurement. Also, the red LED may be repeatedly turned on and off at intervals of 1 second to indicate malfunction.

The plurality of sensors 160 may be arranged to be in contact with the finger of the user. The plurality of sensors 160 may be respectively located in the plurality of openings 145 of the inner electrode 140 and protrude from a surface of the inner electrode 140. The plurality of sensors 160 may be configured to obtain a plurality of first wavelength PPG signals, a plurality of second wavelength PPG signals, and a plurality of third wavelength PPG signals at different locations. Each sensor 160 may include a photoelectric conversion device, and a first wavelength light source, a second wavelength light source, and a third wavelength light source, which emit light of different wavelengths.

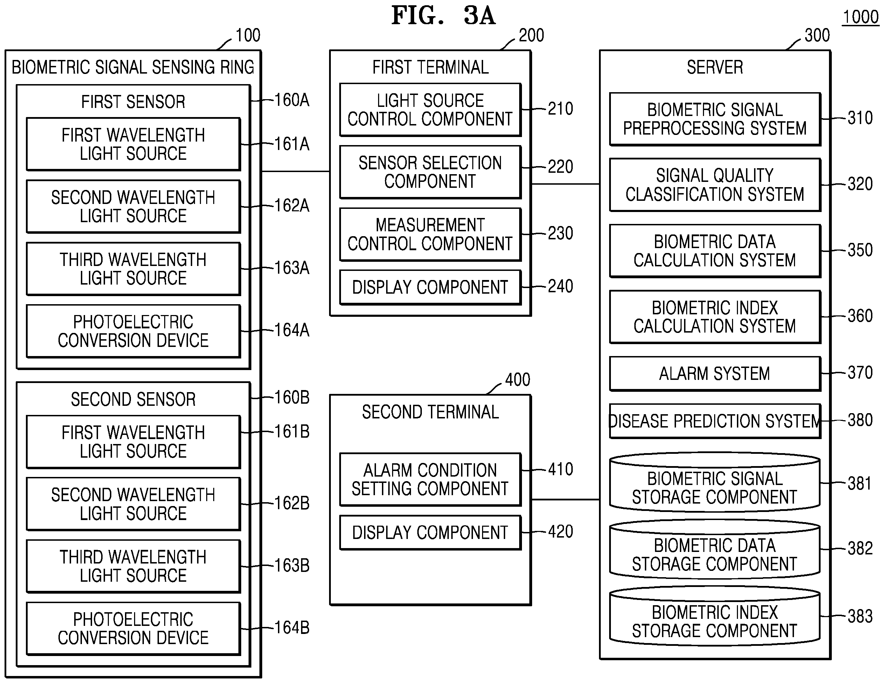

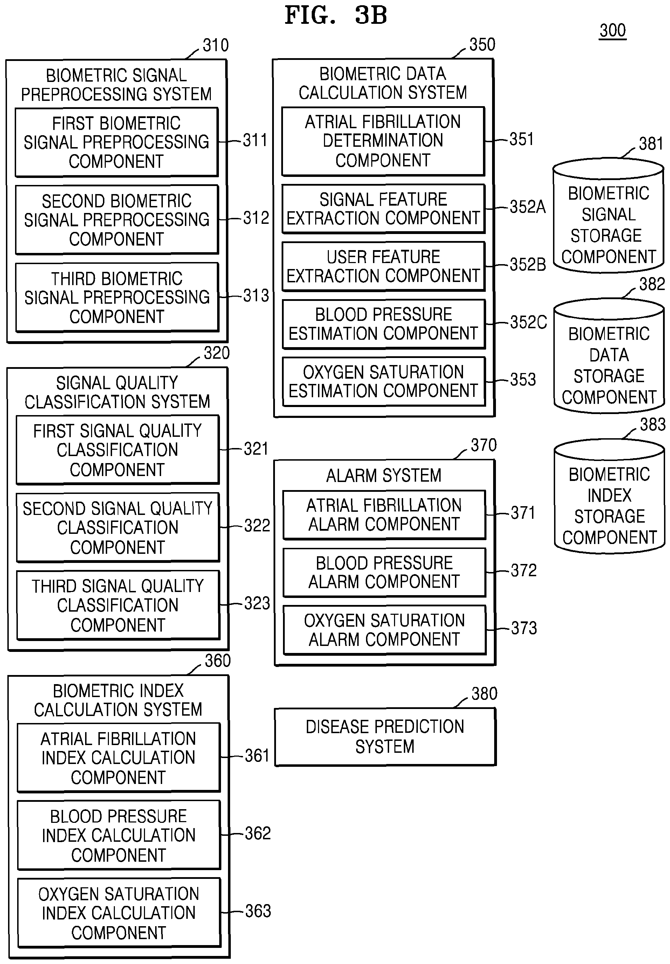

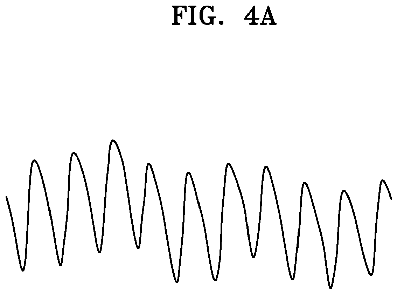

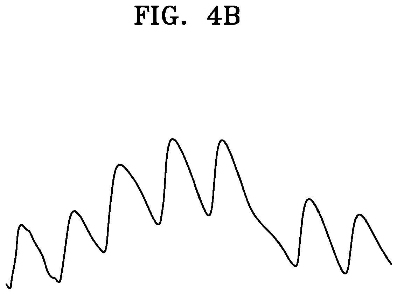

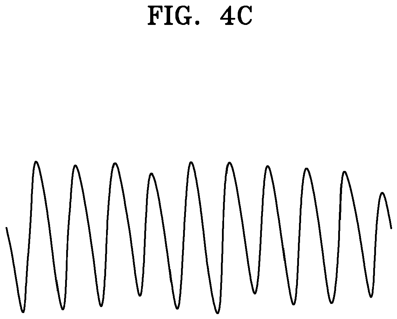

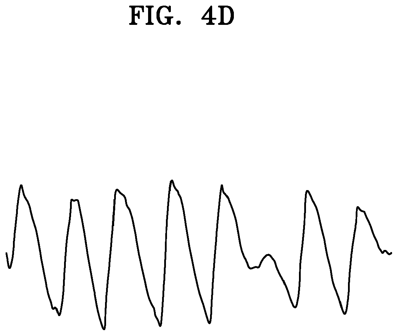

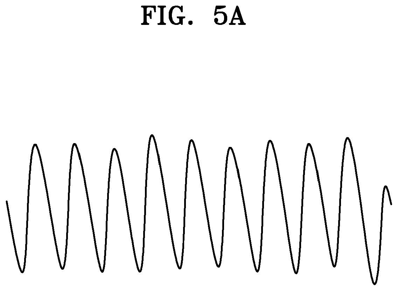

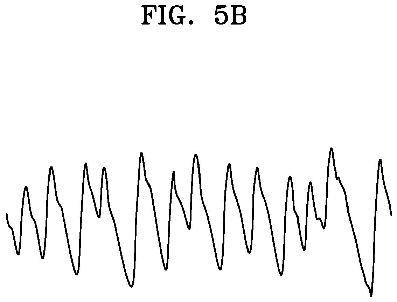

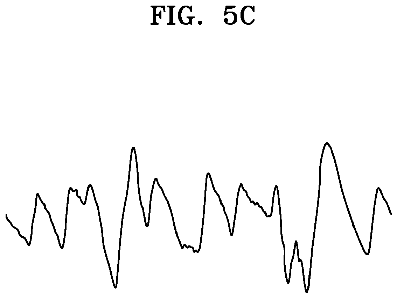

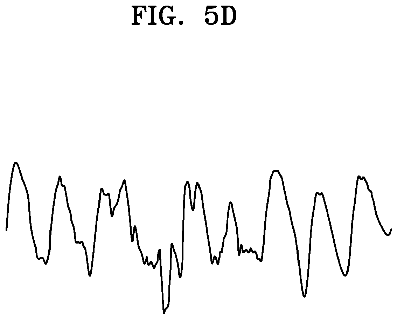

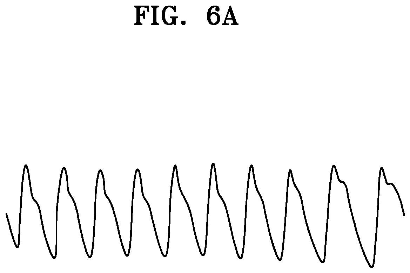

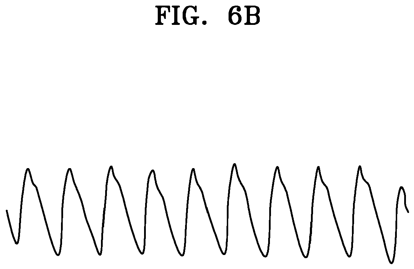

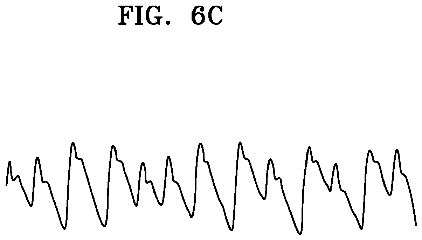

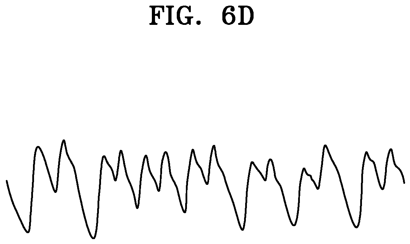

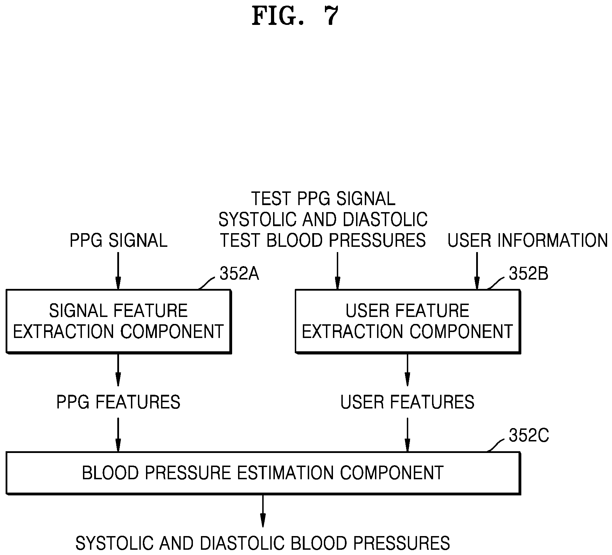

According to some embodiments, although not illustrated in FIGS. 1 and 2, the biometric signal sensing ring 100 may further include an acceleration sensor arranged between the outer electrode 130 and the inner electrode 140. FIG. 3A is a block diagram of a biometric data monitoring platform 1000 using a biometric signal sensing ring, according to an embodiment of the present disclosure. FIG. 3B is a block diagram of a server 300 according to an embodiment of the present disclosure. FIGS. 4A and 4B are graphs showing examples of first wavelength photoplethysmography (PPG) signals before preprocessing. FIGS. 4C and 4D are graphs showing examples of the first wavelength PPG signals of FIGS. 4A and 4B after preprocessing, respectively. FIGS. 5A and 5B are graphs showing examples of first wavelength PPG signals classified as having good quality. FIGS. 5C and 5D are graphs showing examples of first wavelength PPG signals classified as having bad quality. FIGS. 6A and 6B are graphs showing examples of first wavelength PPG signals not determined as atrial fibrillation. FIGS. 6C and 6D are graphs showing examples of first wavelength PPG signals determined as atrial fibrillation. FIG. 7 is a block diagram of a signal feature extraction component 352A, a user feature extraction component 352B, and a blood pressure estimation component 352C.

Referring to FIGS. 3A, 3B, 4A to 4D, 5A to 5D, 6A to 6D, and 7, the biometric data monitoring platform 1000 using a biometric signal sensing ring may include the biometric signal sensing ring 100, a first terminal 200, the server 300, and a second terminal 400.

The biometric signal sensing ring 100 may measure a biometric signal including ECG and a PPG signal. The biometric signal sensing ring 100 may include a plurality of sensors, for example, a first sensor 160A and a second sensor 160B. In FIG. 3A, the biometric signal sensing ring 100 includes two sensors, but the number of sensors included in the biometric signal sensing ring 100 is not limited to two. According to some embodiments, although not shown in FIG. 3A, the biometric signal sensing ring 100 may further include an acceleration sensor.

The first sensor 160A may include a first wavelength light source 161A, a second wavelength light source 162A, a third wavelength light source 163A, and a photoelectric conversion device 164A. The first sensor 160A may detect a first wavelength PPG signal by using the first wavelength light source 161A and the photoelectric conversion device 164A. The first sensor 160A may detect a second wavelength PPG signal by using the second wavelength light source 162A and the photoelectric conversion device 164A. The first sensor 160A may detect a third wavelength PPG signal by using the third wavelength light source 163A and the photoelectric conversion device 164A. The first wavelength light source 161A may include, for example, a green LED, the second wavelength light source 162A may include, for example, an infrared LED, the third wavelength light source 163A may include, for example, a red LED, and the photoelectric conversion device 164A may include a photodiode.

The second sensor 160B may include a first wavelength light source 161B, a second wavelength light source 162B, a third wavelength light source 163B, and a photoelectric conversion device 164B. The second sensor 160B may detect the first wavelength PPG signal by using the first wavelength light source 161B and the photoelectric conversion device 164B. The second sensor 160B may detect the second wavelength PPG signal by using the second wavelength light source 162B and the photoelectric conversion device 164B. The second sensor 160B may detect the third wavelength PPG signal by using the third wavelength light source 163B and the photoelectric conversion device 164B. The first wavelength light source 161B may include, for example, a green LED, the second wavelength light source 162B may include, for example, an infrared LED, the third wavelength light source 163B may include, for example, a red LED, and the photoelectric conversion device 164B may include a photodiode.

The acceleration sensor may detect movement of the user by measuring acceleration of the biometric signal sensing ring 100.

The first terminal 200 may be used by the user. The first terminal 200 may include, for example, a smartphone or a tablet personal computer (PC). The first terminal 200 may be connected to the biometric signal sensing ring 100 wirelessly or via wires. For example, the first terminal 200 may be connected to the biometric signal sensing ring 100 by using Bluetooth or Wi-Fi. The first terminal 200 may be connected to the server 300 wirelessly or via wires. For example, the first terminal 200 may be connected to the server 300 via Wi-Fi or mobile telecommunication technology such as 3rd generation (3G), long-term evolution (LTE), or 5th generation (5G).

The first terminal 200 may include a light source control component 210, a sensor selection component 220, a measurement control component 230, and a display component 240. An application may be installed in the first terminal 200. The light source control component 210, the sensor selection component 220, the measurement control component 230, and the display component 240 may be realized by the application. Alternatively, the first terminal 200 may access a website, and the light source control component 210, the sensor selection component 220, the measurement control component 230, and the display component 240 may be realized by the website.

The light source control component 210 may control the first wavelength light source 161A, the second wavelength light source 162A, and the third wavelength light source 163A of the first sensor 160A, and the first wavelength light source 161B, the second wavelength light source 162B, and the third wavelength light source 163B of the second sensor 160B, such that a direct current (DC) component of each of first wavelength test PPG signals received from the first sensor 160A and the second sensor 160B is within a first predetermined range, a DC component of each of second wavelength test PPG signals received from the first sensor 160A and the second sensor 160B is within a second predetermined range, and a DC component of each of third wavelength test PPG signals received from the first sensor 160A and the second sensor 160B is within a third predetermined range.

After the light source control component 210 controls the first wavelength light source 161A, the second wavelength light source 162A, and the third wavelength light source 163A of the first sensor 160A, and the first wavelength light source 161B, the second wavelength light source 162B, and the third wavelength light source 163B of the second sensor 160B, the sensor selection component 220 may select one of the first sensor 160A and the second sensor 160B as a sensor for measuring a first wavelength PPG signal, a second wavelength PPG signal, and a third wavelength PPG signal afterwards. The sensor selection component 220 may select, from among the first sensor 160A and the second sensor 160B, a sensor that measures a combination of a first wavelength test PPG signal, a second wavelength test PPG signal, and a third wavelength test PPG signal, which has the highest signal quality, from among the two first wavelength test PPG signals, the two second wavelength test PPG signals, and the two third wavelength test PPG signals from the first sensor 160A and the second sensor 160B. Signal quality may be evaluated by at least one of the magnitude of an acceleration signal, a signal-to-noise ratio (SNR), and a ratio of a DC component size to an alternating current (AC) component size. The signal quality may be high when the magnitude of the acceleration signal is low, the SNR is high, and the ratio of a DC component size to an AC component size is high. Then, the sensor selected by the sensor selection component 220 may measure the first wavelength PPG signal, the second wavelength PPG signal, and the third wavelength PPG signal.

The controlling of the first wavelength light source 161A, the second wavelength light source 162A, and the third wavelength light source 163A of the first sensor 160A, and the first wavelength light source 161B, the second wavelength light source 162B, and the third wavelength light source 163B of the second sensor 160B by the light source control component 210, and the selecting of a sensor by the sensor selection component 220 may be sequentially performed. In other words, the sensor selection component 220 may select a sensor after the light source control component 210 controls the first wavelength light source 161A, the second wavelength light source 162A, and the third wavelength light source 163A of the first sensor 160A, and the first wavelength light source 161B, the second wavelength light source 162B, and the third wavelength light source 163B of the second sensor 160B.

The controlling of the first wavelength light source 161A, the second wavelength light source 162A, and the third wavelength light source 163A of the first sensor 160A, and the first wavelength light source 161B, the second wavelength light source 162B, and the third wavelength light source 163B of the second sensor 160B by the light source control component 210, and the selecting of a sensor by the sensor selection component 220 may be periodically performed. The biometric signal sensing ring 100 may move or rotate on a finger, and thus, the light source control component 210 may periodically control a light source and the sensor selection component 220 may periodically select a sensor.

The measurement control component 230 may select a measurement mode or start or end measurement, according to the user's input. For example, the user may select the measurement mode to be one of a self-check mode and a background mode by using the measurement control component 230. In the self-check mode, the user may start or end the measurement by using the measurement control component 230. In the background mode, the measurement starts and continues regardless of the user's input. In the background mode, the user may set or change a measurement cycle. According to some embodiments, the measurement control component 230 may be included in the biometric signal sensing ring 100. In other words, the user may select the measurement mode, or may start or end the measurement by using the measurement control component 230 of the biometric signal sensing ring 100. According to some embodiments, the measurement control component 230 may be included in the server 300. In other words, a service provider may select the measurement mode, or may start or end the measurement by using the measurement control component 230 of the server 300.

The display component 240 may display at least one of a biometric signal measured by the biometric signal sensing ring 100 and stored in a biometric signal storage component 381, a measurement time of the biometric signal stored in the biometric signal storage component 381, a signal quality classification result obtained by a signal quality classification system 320 of the server 300 and stored in a biometric data storage component 382 of the server 300, biometric data calculated by a biometric data calculation system 350 of the server 300 and stored in the biometric data storage component 382 of the server 300, a biometric index calculated by a biometric index calculation system 360 of the server 300 and stored in a biometric index storage component 383, and a disease occurrence probability calculated by a disease prediction system 380.

Here, the biometric signal may include at least one of ECG, a first wavelength PPG signal, a second wavelength PPG signal, and a third wavelength PPG signal. Also, the biometric data may include at least one of an atrial fibrillation determination result, blood pressure, and oxygen saturation. Also, the biometric index may include at least one of an atrial fibrillation index, a blood pressure index, and an oxygen saturation index.

The server 300 may include a biometric signal preprocessing system 310, the signal quality classification system 320, the biometric data calculation system 350, the biometric index calculation system 360, an alarm system 370, the disease prediction system 380, the biometric signal storage component 381, the biometric data storage component 382, and the biometric index storage component 383.

The biometric signal preprocessing system 310 may preprocess the biometric signal received by the server 300 from the selected sensor of the biometric signal sensing ring 100 through the first terminal 200. The biometric signal preprocessing system 310 may include a first biometric signal preprocessing component 311, a second biometric signal preprocessing component 312, and a third biometric signal preprocessing component 313.

The first biometric signal preprocessing component 311 may preprocess the first wavelength PPG signal. For example, a low-pass filter, a high-pass filter, and normalization may be used to preprocess the first wavelength PPG signal. FIGS. 4A and 4B are graphs showing examples of the first wavelength PPG signals before preprocessing. FIGS. 4C and 4D are graphs showing examples of the first wavelength PPG signals of FIGS. 4A and 4B after preprocessing, respectively.

The second biometric signal preprocessing component 312 may preprocess the second wavelength PPG signal. For example, a low-pass filter, a high-pass filter, and normalization may be used to preprocess the second wavelength PPG signal.

The third biometric signal preprocessing component 313 may preprocess the second wavelength PPG signal and the third wavelength PPG signal. For example, a low-pass filter, a high-pass filter, and normalization may be used to preprocess the second wavelength PPG signal and the third wavelength PPG signal.

According to some embodiments, the biometric signal preprocessing system 310 may further include a fourth biometric signal preprocessing component, and the fourth biometric signal preprocessing component may preprocess an acceleration signal received by the server 300 from the acceleration sensor of the biometric signal sensing ring 100 through the first terminal 200.

The signal quality classification system 320 may classify signal quality of the biometric signal preprocessed by the biometric signal preprocessing system 310 as being good or bad. The signal quality classification system 320 may include a first signal quality classification component 321, a second signal quality classification component 322, and a third signal quality classification component 323.

The first signal quality classification component 321 may classify signal quality of the first wavelength PPG signal preprocessed by the first biometric signal preprocessing component 311 as being good or bad. FIGS. 5A and 5B are graphs showing examples of the first wavelength PPG signals classified as having good quality. FIGS. 5C and 5D are graphs showing examples of the first wavelength PPG signals classified as having bad quality. According to some embodiments, the first signal quality classification component 321 may refer to the acceleration signal to classify the signal quality of the first wavelength PPG signal as being one of good and bad.

According to some embodiments, the first signal quality classification component 321 may classify quality of the first wavelength PPG signal as being good or bad by using a deep learning model. The deep learning model used by the first signal quality classification component 321 may include a convolution neural network (CNN), a long short-term memory (LSTM), a fully-connected network (FCN), an encoder, a decoder, or a combination thereof. A method of classifying signal quality is not limited to the method described in the present specification.

The second signal quality classification component 322 may classify signal quality of the second wavelength PPG signal preprocessed by the second biometric signal preprocessing component 312 as being good or bad. According to some embodiments, the second signal quality classification component 322 may refer to the acceleration signal to classify the signal quality of the second wavelength PPG signal as being one of good and bad. According to some embodiments, the second signal quality classification component 322 may classify quality of the second wavelength PPG signal as being good or bad by using a deep learning model. The deep learning model used by the second signal quality classification component 322 may include a CNN, an LSTM, an FCN, an encoder, a decoder, or a combination thereof. A method of classifying signal quality is not limited to the method described in the present specification.

The third signal quality classification component 323 may classify the signal quality of the preprocessed second wavelength PPG signal and third wavelength PPG signal as good or bad. According to some embodiments, the third signal quality classification component 323 may refer to the acceleration signal to classify the signal quality of the third wavelength PPG signal as being one of good and bad. According to some embodiments, the third signal quality classification component 323 may classify quality of the second wavelength PPG signal and the third wavelength PPG signal as being good or bad by using a deep learning model. The deep learning model used by the third signal quality classification component 323 may include a CNN, an LSTM, an FCN, an encoder, a decoder, or a combination thereof. A method of classifying signal quality is not limited to the method described in the present specification.

It may be determined that the biometric data calculated from the biometric signal of which the signal quality is classified as being bad by the signal quality classification system 320 is unreliable, and the biometric data may not be displayed by the display component 240 of the first terminal 200. It may be determined that the biometric data calculated from the biometric signal of which the quality is classified as being good is reliable, and the biometric data may be displayed by the display component 240 of the first terminal 200.

According to an alternative embodiment, the biometric data calculated from the biometric signal may be displayed by the display component 240 of the first terminal 200 together with the signal quality classification result, regardless of the signal quality classification result. Also, the biometric data calculated from the biometric signal may be displayed by a display component 420 of the second terminal 400 together with the signal quality classification result, regardless of the signal quality classification result.

The biometric data calculation system 350 may calculate the biometric data from the biometric signal. The biometric signal may include at least one of ECG, a first wavelength PPG signal, a second wavelength PPG signal, and a third wavelength PPG signal. The biometric data may include at least one of an atrial fibrillation determination result, blood pressure, and oxygen saturation. The biometric data calculation system 350 may include an atrial fibrillation determination component 351, the signal feature extraction component 352A, the user feature extraction component 352B, the blood pressure estimation component 352C, and an oxygen saturation estimation component 353.

The atrial fibrillation determination component 351 may determine whether atrial fibrillation has occurred from the first wavelength PPG signal by using a deep learning model. FIGS. 6A and 6B are graphs showing examples of the first wavelength PPG signals not determined as atrial fibrillation. FIGS. 6C and 6D are graphs showing examples of the first wavelength PPG signals determined as atrial fibrillation.

The deep learning model used by the atrial fibrillation determination component 351 may include a CNN, an LSTM, an FCN, an encoder, a decoder, or a combination thereof. The deep learning model may be trained by using supervised learning. Data augmentation may be used to secure sufficient data sets required for supervised learning.

As shown in FIG. 7, the signal feature extraction component 352A may extract a PPG feature from the second wavelength PPG signal by using a first deep learning model. The user feature extraction component 352B may extract a user feature from user information, a test PPG signal, and systolic and diastolic test blood pressure, by using a second deep learning model. The test PPG signal may be measured by using a second wavelength light source and a photoelectric conversion device of a selected sensor of the biometric signal sensing ring 100. The systolic and diastolic test blood pressure may be measured by using a general blood pressure gauge, together with the test PPG signal. The user information may include at least one of the user's age, weight, height, and gender. The blood pressure estimation component 352C may estimate systolic and diastolic blood pressure from the PPG feature and the user feature, by using a third deep learning model. The test PPG signal, the systolic and diastolic test blood pressure, and the user information may be periodically updated.

The first deep learning model, the second deep learning model, and the third deep learning model may each include a CNN, an LSTM, an FCN, an encoder, a decoder, or a combination thereof. The first deep learning model, the second deep learning model, and the third deep learning model may be trained by using supervised learning. Data augmentation may be used to secure sufficient data sets required for supervised learning.

The oxygen saturation estimation component 353 may estimate the oxygen saturation from the second wavelength PPG signal and the third wavelength PPG signal. Various methods may be used to estimate the oxygen saturation.

For example, a value of R may be first calculated according to Equation 1 below.

[Equation 1]

$$R = ((AC_R)/(DC_R))/((AC_{IR})/(DC_{IR}))$$

Here, $AC_R$ denotes the size of an AC component of the third wavelength PPG signal, $DC_R$ denotes the size of a DC component of the third wavelength PPG signal, $AC_{IR}$ denotes the size of an AC component of the second wavelength PPG signal, and $DC_{IR}$ denotes the size of a DC component of the second wavelength PPG signal.

Then, oxygen saturation may be calculated from the value of R, according to Equation 2 below.

[Equation 2]

$$\text{Oxygen Saturation} = C_0 - C_1 R$$

$C_0$ and $C_1$ are constants that may be determined by using linear regression.

In Equation 2, the oxygen saturation is represented as a primary expression for the value of R, but the oxygen saturation may be represented as a polynomial expression of a degree greater than 1 for the value of R, for example, a quadratic or cubic expression. A method of estimating oxygen saturation is not limited to the method described in the present specification.

The biometric index calculation system 360 may calculate a biometric index from the signal quality classification result generated by the signal quality classification system 320 and the biometric data calculated by the biometric data calculation system 350. Here, the biometric index may include at least one of an atrial fibrillation index, a blood pressure index, and an oxygen saturation index. The biometric index calculation system 360 may include an atrial fibrillation index calculation component 361, a blood pressure index calculation component 362, and an oxygen saturation index calculation component 363.

The atrial fibrillation index calculation component 361 may calculate an atrial fibrillation index from a first signal quality classification result generated by the first signal quality classification component 321 and an atrial fibrillation determination result generated by the atrial fibrillation determination component 351. The atrial fibrillation index may be defined based on a time when the quality of the first wavelength PPG signal is classified as being good by the first signal quality classification component 321, and a time when atrial fibrillation is determined to have occurred by the atrial fibrillation determination component 351 among the time when the quality of the first wavelength PPG signal is classified as being good by the first signal quality classification component 321. For example, the atrial fibrillation index may be defined by a ratio of the time when the quality of the first wavelength PPG signal is classified as being good by the first signal quality classification component 321 to the time when the atrial fibrillation is determined to have occurred by the atrial fibrillation determination component 351 among the time when the quality of the first wavelength PPG signal is classified as being good by the first signal quality classification component 321. In other words, the atrial fibrillation index may denote a ratio of the time when the atrial fibrillation has occurred among a time when a reliable atrial fibrillation determination result is obtainable.

The blood pressure index calculation component 362 may calculate a blood pressure index from a second signal quality classification result generated by the second signal quality classification component 322 and blood pressure estimated by the blood pressure estimation component 352C. The blood pressure index may be defined based on a time when the quality of the second wavelength PPG signal is classified as being good by the second signal quality classification component 322, and a time when systolic blood pressure is outside a first normal range or diastolic blood pressure is outside a second normal range among the time when the quality of the second wavelength PPG signal is classified as being good by the second signal quality classification component 322. For example, the blood pressure index may be defined by a ratio of the time when the quality of the second wavelength PPG signal is classified as being good by the second signal quality classification component 322 to the time when the systolic blood pressure is outside the first normal range or the diastolic blood pressure is outside the second normal range among the time when the quality of the second wavelength PPG signal is classified as being good by the second signal quality classification component 322. In other words, the blood pressure index may denote a ratio of a time when abnormal blood pressure occurs among a time when a reliable blood pressure estimation result is obtainable.

The oxygen saturation index calculation component 363 may calculate the oxygen saturation index from a third signal quality classification result generated by the third signal quality classification component 323 and oxygen saturation estimated by the oxygen saturation estimation component 353. The oxygen saturation index may be defined based on a time when the quality of the second wavelength PPG signal and the third wavelength PPG signal is classified as being good by the third signal quality classification component 323, and a time when the oxygen saturation estimated by the oxygen saturation estimation component 353 is outside a normal range among the time when the quality of the second wavelength PPG signal and the third wavelength PPG signal is classified as being good by the third signal quality classification component 323. For example, the oxygen saturation index may be defined by a ratio of the time when the quality of the second wavelength PPG signal and the third wavelength PPG signal is classified as being good by the third signal quality classification component 323 to the time when the oxygen saturation estimated by the oxygen saturation estimation component 353 is outside the normal range among the time when the quality of the second wavelength PPG signal and the third wavelength PPG signal is classified as being good by the third signal quality classification component 323. In other words, the oxygen saturation index may denote the ratio of the time when oxygen saturation is outside the normal range to the time when reliable oxygen saturation is obtainable.

The alarm system 370 may transmit an alarm to at least one of the first terminal 200 and the second terminal 400 when at least one of the biometric signal, the biometric data, and the biometric index satisfies an alarm condition pre-set by an alarm condition setting component 410. The alarm system 370 may include an atrial fibrillation alarm component 371, a blood pressure alarm component 372, and an oxygen saturation alarm component 373.

The atrial fibrillation alarm component 371 may transmit an alarm to at least one of the first terminal 200 and the second terminal 400 when an atrial fibrillation determination result obtained by the atrial fibrillation determination component 351 satisfies the alarm condition set by the alarm condition setting component 410 of the second terminal 400.

The blood pressure alarm component 372 may transmit an alarm to at least one of the first terminal 200 and the second terminal 400 when blood pressure estimated by the blood pressure estimation component 352C satisfies the alarm condition set by the alarm condition setting component 410 of the second terminal 400.

The oxygen saturation alarm component 373 may transmit an alarm to at least one of the first terminal 200 and the second terminal 400 when oxygen saturation estimated by the oxygen saturation estimation component 353 satisfies the alarm condition set by the alarm condition setting component 410 of the second terminal 400.

The disease prediction system 380 may calculate a disease occurrence probability from a change in at least one of the biometric signal, e.g., the first wavelength PPG signal, the second wavelength PPG signal, and the third wavelength PPG signal, the biometric data, e.g., the atrial fibrillation determination result, the blood pressure, and the oxygen saturation, and the biometric index, e.g., the atrial fibrillation index, the blood pressure index, and the oxygen saturation index, over time. The disease prediction system 380 may use a deep learning model.

The biometric signal storage component 381 may store the ECG, the first wavelength PPG signal, the second wavelength PPG signal, and the third wavelength PPG signal received by the server 300 from the biometric signal sensing ring 100 through the first terminal 200. The biometric signal storage component 381 may further store measurement times of the ECG, the first wavelength PPG signal, the second wavelength PPG signal, and the third wavelength PPG signal. The biometric signal storage component 381 may further store the first wavelength PPG signal, the second wavelength PPG signal, and the third wavelength PPG signal preprocessed by the biometric signal preprocessing system 310. The biometric signal storage component 381 may further store the acceleration signal received by the server 300 from the biometric signal sensing ring 100 through the first terminal 200. The biometric signal storage component 381 may further store the acceleration signal preprocessed by the biometric signal preprocessing system 310.

The biometric data storage component 382 may store the biometric data, such as the atrial fibrillation determination result generated by the atrial fibrillation determination component 351, the blood pressure estimated by the blood pressure estimation component 352C, and the oxygen saturation estimated by the oxygen saturation estimation component 353. The biometric data storage component 382 may store the first signal quality classification result generated by the first signal quality classification component 321, the second signal quality classification result generated by the second signal quality classification component 322, and the third signal quality classification result generated by the third signal quality classification component 323. The biometric index storage component 383 may store the biometric index, for example, the atrial fibrillation index calculated by the atrial fibrillation index calculation component 361, the blood pressure index calculated by the blood pressure index calculation component 362, and the oxygen saturation index calculated by the oxygen saturation index calculation component 363.

The second terminal 400 may be connected to the server 300 wirelessly or via wires. For example, the second terminal 400 may be connected to the server 300 via Wi-Fi or mobile telecommunication technology such as 3G, LTE, or 5G. The second terminal 400 may be used by a doctor. The second terminal 400 may include, for example, a smartphone, a tablet PC, a computer, or a laptop computer.

The second terminal 400 may include the alarm condition setting component 410 and the display component 420. The second terminal 400 may access a website or an application may be installed in the second terminal 400. The alarm condition setting component 410 and the display component 420 may be realized by using the website or the application.

The doctor may set the alarm condition by using the alarm condition setting component 410. For example, the atrial fibrillation alarm component 371 may be set to transmit an alarm to at least one of the first terminal 200 and the second terminal 400 when atrial fibrillation continues for 10 minutes or more. Also, for example, the blood pressure alarm component 372 may be set to transmit an alarm to at least one of the first terminal 200 and the second terminal 400 when systolic blood pressure exceeding 140 mmHg or less than 90 mmHg or diastolic blood pressure exceeding 90 mmHg or less than 60 mmHg continues for 10 minutes or more. Also, for example, the oxygen saturation alarm component 373 may be set to transmit an alarm to at least one of the first terminal 200 and the second terminal 400 when oxygen saturation less than 90 % continues for 10 minutes or more.

According to some embodiments, the alarm condition setting component 410 may be included in the server 300. In other words, a service provider may set the alarm condition by using the alarm condition setting component 410 of the server 300. According to some embodiments, the alarm condition setting component 410 may be included in the first terminal 200. In other words, a user may set the alarm condition by using the alarm condition setting component 410 of the first terminal 200.

The display component 420 may display at least one of the biometric signal measured by the biometric signal sensing ring 100 and stored in the biometric signal storage component 381 of the server 300, the measurement time of the biometric signal stored in the biometric signal storage component 381 of the server 300, the preprocessed biometric signal stored in the biometric signal storage component 381, the signal quality classification result obtained by the signal quality classification system 320 of the server 300 and stored in the biometric data storage component 382 of the server 300, the biometric data calculated by the biometric data calculation system 350 of the server 300 and stored in the biometric data storage component 382 of the server 300, the biometric index calculated by the biometric index calculation system 360 of the server 300 and stored in the biometric index storage component 383, and the disease occurrence probability calculated by the disease prediction system 380.

The embodiments in the present disclosure are not intended to limit the technical ideas of the present disclosure but to describe the present disclosure, and the scope of the technical ideas of the present disclosure is not limited by these embodiments. The scope of protection of the present disclosure should be interpreted by the following claims, and all technical ideas within the scope equivalent thereto should be construed as being included in the scope of the present disclosure.

**Claims**

1. A biometric data monitoring platform using a biometric signal sensing ring, the platform comprising a server, wherein the server comprises:

   a signal quality classification system including a first signal quality classification component configured to classify signal quality of a first wavelength photoplethysmography (PPG) signal as being good or bad;
   a biometric data calculation system including an atrial fibrillation determination component configured to determine whether atrial fibrillation has occurred, from the first wavelength PPG signal by using a deep learning model; and
   a biometric index calculation system including an atrial fibrillation index calculation component configured to calculate an atrial fibrillation index from a first signal quality classification result generated by the first signal quality classification component and an atrial fibrillation determination result generated by the atrial fibrillation determination component,
   wherein the atrial fibrillation index is defined by a ratio of a time when quality of the first wavelength PPG signal is classified as being good by the first signal quality classification component to a time when the quality of the first wavelength PPG signal is classified as being good by the first signal quality classification component and the atrial fibrillation is determined to have occurred by the atrial fibrillation determination component.

2. The biometric data monitoring platform of claim 1, wherein the signal quality classification system further comprises a second signal quality classification component configured to classify signal quality of a second wavelength PPG signal as being good or bad, and
   the biometric data calculation system further comprises:

   a signal feature extraction component configured to extract, from the second wavelength PPG signal, a PPG feature by using a first deep learning model;
   a user feature extraction component configured to extract a user feature by using a second deep learning model, from user information, a test PPG signal measured by using the biometric signal sensing ring, and systolic and diastolic test blood pressure measured simultaneously with the test PPG signal by using a general blood pressure gauge; and
   a blood pressure estimation component configured to estimate systolic and diastolic blood pressure from the PPG feature and the user feature by using a third deep learning model.

3. The biometric data monitoring platform of claim 2, wherein the biometric index calculation system further comprises a blood pressure index calculation component configured to calculate a blood pressure index from blood pressure estimated by the blood pressure estimation component and a second signal quality classification result generated by the second signal quality classification component, and
   the blood pressure index is defined by a ratio of a time when quality of the second wavelength PPG signal is classified as being good by the second signal quality classification component to a time when the quality of the second wavelength PPG signal is classified as being good by the second signal quality classification component and the systolic blood pressure is outside a first normal range or the diastolic blood pressure is outside a second normal range.

4. The biometric data monitoring platform of claim 1, wherein the signal quality classification system further comprises a third signal quality classification component configured to classify quality of a second wavelength PPG signal and a third wavelength PPG signal as being good or bad, and
the biometric data calculation system further comprises an oxygen saturation estimation component configured to estimate oxygen saturation from the second wavelength PPG signal and the third wavelength PPG signal.

5. The biometric data monitoring platform of claim 4, wherein the biometric index calculation system further comprises an oxygen saturation index calculation component configured to calculate an oxygen saturation index from oxygen saturation estimated by the oxygen saturation estimation component and a third signal quality classification result generated by the third signal quality classification component, and
the oxygen saturation index is defined by a ratio of a time when the quality of the second wavelength PPG signal and the third wavelength PPG signal is classified as being good by the third signal quality classification component to a time when the quality of the second wavelength PPG signal and the third wavelength PPG signal are classified as being good by the third signal quality classification component and the oxygen saturation is outside a normal range.

6. The biometric data monitoring platform of claim 1, wherein the first wavelength PPG signal, the second wavelength PPG signal, and the third wavelength PPG signal are measured by using the biometric signal sensing ring and the server receives the first wavelength PPG signal, the second wavelength PPG signal, and the third wavelength PPG signal from the biometric signal sensing ring through a terminal,

the biometric signal sensing ring comprises a plurality of sensors configured to simultaneously measure a plurality of first wavelength PPG signals, a plurality of second wavelength PPG signals, and a plurality of third wavelength PPG signals at different locations, respectively, and
each of the plurality of sensors comprises a first wavelength light source, a second wavelength light source, a third wavelength light source, and a photoelectric conversion device.

7. The biometric data monitoring platform of claim 6, wherein the terminal comprises a light source control component configured to control the first wavelength light source, the second wavelength light source, and the third wavelength light source of each of the plurality of sensors such that a direct current (DC) component of each of first wavelength test PPG signals measured by using the plurality of sensors is within a first predetermined range, a DC component of each of a plurality of second wavelength test PPG signals measured by using the plurality of sensors is within a second predetermined range, and a DC component of each of a plurality of third wavelength test PPG signals measured by using the plurality of sensors is within a third predetermined range.

8. The biometric data monitoring platform of claim 7, wherein the terminal further comprises a sensor selection component configured to select, from among the plurality of sensors, as a sensor for measuring the first wavelength PPG signal, the second wavelength PPG signal, and the third wavelength PPG signal, a sensor that measures a combination of a first wavelength test PPG signal, a second wavelength test PPG signal, and a third wavelength test PPG signal, which has highest signal quality, from among the plurality of first wavelength test PPG signals, the plurality of second wavelength test PPG signals, and the plurality of third wavelength test PPG signals.

9. The biometric data monitoring platform of claim 8, wherein signal quality of the plurality of first wavelength test PPG signals, the plurality of second wavelength test PPG signals, and the plurality of third wavelength test PPG signals is evaluated by at least one of magnitude of an acceleration signal, a signal-to-noise ratio, and a ratio of a DC component size to an alternating current (AC) component size.

10. The biometric data monitoring platform of claim 8, wherein the controlling of the first wavelength light source, the second wavelength light source, and the third wavelength light source of each of the plurality of sensors by the light source control component and the selecting of the sensor by the sensor selection component are sequentially performed, and
the controlling of the first wavelength light source, the second wavelength light source, and the third wavelength light source of each of the plurality of sensors by the light source control component and the selecting of the sensor by the sensor selection component are periodically performed.

11. A biometric data monitoring platform using a biometric signal sensing ring, the platform comprising a terminal, wherein the terminal is configured to receive a photoplethysmography (PPG) signal from a biometric signal sensing ring configured to measure the PPG signal, and transmit the PPG signal to a server,

the biometric signal sensing ring comprises a plurality of sensors configured to simultaneously measure a plurality of PPG signals at different locations, respectively, and
each of the plurality of sensors comprises a light source and a photoelectric conversion device.

12. The biometric data monitoring platform of claim 11, wherein the terminal comprises a light source control component configured to control the light source of each of the plurality of sensors such that a direct current (DC) component of each of a plurality of test PPG signals measured by using the plurality of sensors is within a predetermined range.

13. The biometric data monitoring platform of claim 12, wherein the terminal further comprises a sensor selection component configured to select, from among the plurality of sensors, as a sensor for measuring the PPG signal, a sensor that measures a test PPG signal with highest signal quality.

14. The biometric data monitoring platform of claim 13, wherein signal quality of the plurality of test PPG signals is evaluated by at least one of magnitude of an acceleration signal, a signal-to-noise ratio, and a ratio of a DC component size to an alternating current (AC) component size.

15. The biometric data monitoring platform of claim 14, wherein the controlling of the light source of each of the plurality of sensors by the light source control component and the selecting of the sensor by the sensor selection component are sequentially performed, and
the controlling of the light source of each of the plurality of sensors by the light source control component and the selecting of the sensor by the sensor selection component are periodically performed.

16. A biometric data monitoring platform using a biometric signal sensing ring, the platform comprising a terminal, wherein the terminal is configured to receive a first wavelength PPG signal, a second wavelength PPG signal, and a third wavelength PPG signal from the biometric signal sensing ring configured to measure the first wavelength PPG signal, the second wavelength PPG signal, and the third wavelength PPG signal, and transmit the first wavelength PPG signal, the second wavelength PPG signal, and the third wavelength PPG signal to a server,

the biometric signal sensing ring comprises a plurality of sensors configured to simultaneously measure a plurality of first wavelength PPG signals, a plurality of second wavelength PPG signals, and a plurality of third wavelength PPG signals at different locations, respectively, and

each of the plurality of sensors comprises a first wavelength light source, a second wavelength light source, a third wavelength light source, and a photoelectric conversion device.

17. The biometric data monitoring platform of claim 16, wherein the terminal comprises a light source control component configured to control the first wavelength light source, the second wavelength light source, and the third wavelength light source of each of the plurality of sensors such that a direct current (DC) component of each of first wavelength test PPG signals measured by using the plurality of sensors is within a first predetermined range, a DC component of each of a plurality of second wavelength test PPG signals measured by using the plurality of sensors is within a second predetermined range, and a DC component of each of a plurality of third wavelength test PPG signals measured by using the plurality of sensors is within a third predetermined range.

18. The biometric data monitoring platform of claim 17, wherein the terminal further comprises a sensor selection component configured to select, from among the plurality of sensors, as a sensor for measuring the first wavelength PPG signal, the second wavelength PPG signal, and the third wavelength PPG signal, a sensor that measures a combination of a first wavelength test PPG signal, a second wavelength test PPG signal, and a third wavelength test PPG signal, which has highest signal quality, from among the plurality of first wavelength test PPG signals, the plurality of second wavelength test PPG signals, and the plurality of third wavelength test PPG signals.

19. The biometric data monitoring platform of claim 18, wherein signal quality of the plurality of first wavelength test PPG signals, the plurality of second wavelength test PPG signals, and the plurality of third wavelength test PPG signals is evaluated by at least one of magnitude of an acceleration signal, a signal-to-noise ratio, and a ratio of a DC component size to an alternating current (AC) component size.

20. The biometric data monitoring platform of claim 18, wherein the controlling of the first wavelength light source, the second wavelength light source, and the third wavelength light source of each of the plurality of sensors by the light source control component and the selecting of the sensor by the sensor selection component are sequentially performed, and
the controlling of the first wavelength light source, the second wavelength light source, and the third wavelength light source of each of the plurality of sensors by the light source control component and the selecting of the sensor by the sensor selection component are periodically performed.

# FIG. 1

# FIG. 2

# FIG. 3A

**100** — BIOMETRIC SIGNAL SENSING RING

- FIRST SENSOR — 160A
  - FIRST WAVELENGTH LIGHT SOURCE — 161A
  - SECOND WAVELENGTH LIGHT SOURCE — 162A
  - THIRD WAVELENGTH LIGHT SOURCE — 163A
  - PHOTOELECTRIC CONVERSION DEVICE — 164A
- SECOND SENSOR — 160B
  - FIRST WAVELENGTH LIGHT SOURCE — 161B
  - SECOND WAVELENGTH LIGHT SOURCE — 162B
  - THIRD WAVELENGTH LIGHT SOURCE — 163B
  - PHOTOELECTRIC CONVERSION DEVICE — 164B

**200** — FIRST TERMINAL

- LIGHT SOURCE CONTROL COMPONENT — 210
- SENSOR SELECTION COMPONENT — 220
- MEASUREMENT CONTROL COMPONENT — 230
- DISPLAY COMPONENT — 240

**400** — SECOND TERMINAL

- ALARM CONDITION SETTING COMPONENT — 410
- DISPLAY COMPONENT — 420

**1000**

**300** — SERVER

- BIOMETRIC SIGNAL PREPROCESSING SYSTEM — 310
- SIGNAL QUALITY CLASSIFICATION SYSTEM — 320
- BIOMETRIC DATA CALCULATION SYSTEM — 350
- BIOMETRIC INDEX CALCULATION SYSTEM — 360
- ALARM SYSTEM — 370
- DISEASE PREDICTION SYSTEM — 380
- BIOMETRIC SIGNAL STORAGE COMPONENT — 381
- BIOMETRIC DATA STORAGE COMPONENT — 382
- BIOMETRIC INDEX STORAGE COMPONENT — 383

EP 4 403 094 A1

# FIG. 3B

<u>300</u>

FIG. 4A

FIG. 4B

# FIG. 4C

FIG. 4D

FIG. 5A

FIG. 5B

FIG. 5C

FIG. 5D

FIG. 6A

FIG. 6B

FIG. 6C

FIG. 6D

# FIG. 7

PPG SIGNAL

TEST PPG SIGNAL
SYSTOLIC AND DIASTOLIC
TEST BLOOD PRESSURES      USER INFORMATION

352A

352B

| SIGNAL FEATURE EXTRACTION COMPONENT | USER FEATURE EXTRACTION COMPONENT |

PPG FEATURES                    USER FEATURES

352C

| BLOOD PRESSURE ESTIMATION COMPONENT |

SYSTOLIC AND DIASTOLIC BLOOD PRESSURES

## INTERNATIONAL SEARCH REPORT

| International application No. |
| --- |
| **PCT/KR2022/013741** |

### A. CLASSIFICATION OF SUBJECT MATTER

**A61B 5/00**(2006.01)i; **A61B 5/024**(2006.01)i; **A61B 5/021**(2006.01)i; **A61B 5/1455**(2006.01)i; **G16H 50/20**(2018.01)i

According to International Patent Classification (IPC) or to both national classification and IPC

### B. FIELDS SEARCHED

Minimum documentation searched (classification system followed by classification symbols)

A61B 5/00(2006.01); A61B 5/01(2006.01); A61B 5/024(2006.01); A61B 5/026(2006.01); A61B 5/24(2021.01); G06F 19/00(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블(wearable), 반지(ring), 광용적맥파(PPG, photoplethysmography), 신호 품질(signal quality), 심전도(ECG, electrocardiogram), 모니터링(monitoring)

### C. DOCUMENTS CONSIDERED TO BE RELEVANT

| Category* | Citation of document, with indication, where appropriate, of the relevant passages | Relevant to claim No. |
| --- | --- | --- |
| Y | KR 10-2019-0110874 A (BIOMEDILABS CO., LTD.) 01 October 2019 (2019-10-01)<br>See paragraphs [0035]-[0087]; claims 1 and 5; and figure 4. | 11-20 |
| A | | 1-10 |
| Y | KR 10-2015-0062721 A (MOON, Chan Gon) 08 June 2015 (2015-06-08)<br>See claims 1 and 15. | 11-20 |
| Y | KR 10-2019-0113552 A (SAMSUNG ELECTRONICS CO., LTD.) 08 October 2019 (2019-10-08)<br>See paragraphs [0023]-[0047]; and claim 1. | 12-15,17-20 |
| A | KR 10-2017-0091346 A (SAMSUNG ELECTRONICS CO., LTD.) 09 August 2017 (2017-08-09)<br>See entire document. | 1-20 |
| A | KR 10-2018-0056714 A (QUALCOMM INCORPORATED) 29 May 2018 (2018-05-29)<br>See entire document. | 1-20 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 December 2022** | **05 December 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| INTERNATIONAL SEARCH REPORT | International application No. |
|---|---|
| Information on patent family members | **PCT/KR2022/013741** |

| Patent document cited in search report | | | Publication date (day/month/year) | Patent family member(s) | | | Publication date (day/month/year) |
|---|---|---|---|---|---|---|---|
| KR | 10-2019-0110874 | A | 01 October 2019 | EP | 3769666 | A1 | 27 January 2021 |
| | | | | KR | 10-2062646 | B1 | 06 January 2020 |
| | | | | US | 2021-0076951 | A1 | 18 March 2021 |
| | | | | WO | 2019-182258 | A1 | 26 September 2019 |
| KR | 10-2015-0062721 | A | 08 June 2015 | KR | 10-1679728 | B1 | 25 November 2016 |
| | | | | WO | 2015-080360 | A1 | 04 June 2015 |
| KR | 10-2019-0113552 | A | 08 October 2019 | CN | 107224269 | A | 03 October 2017 |
| | | | | CN | 110301911 | A | 08 October 2019 |
| | | | | DE | 102019104798 | A1 | 02 October 2019 |
| | | | | EP | 3222208 | A1 | 27 September 2017 |
| | | | | JP | 2017-170137 | A | 28 September 2017 |
| | | | | KR | 10-2017-0112954 | A | 12 October 2017 |
| | | | | TW | 201733521 | A | 01 October 2017 |
| | | | | US | 10390758 | B2 | 27 August 2019 |
| | | | | US | 10750960 | B2 | 25 August 2020 |
| | | | | US | 2017-0273620 | A1 | 28 September 2017 |
| | | | | US | 2018-0279891 | A1 | 04 October 2018 |
| | | | | US | 2019-0365319 | A1 | 05 December 2019 |
| KR | 10-2017-0091346 | A | 09 August 2017 | EP | 3384800 | A1 | 10 October 2018 |
| | | | | EP | 3384800 | B1 | 24 August 2022 |
| | | | | KR | 10-2464916 | B1 | 08 November 2022 |
| | | | | US | 11324292 | B2 | 10 May 2022 |
| | | | | US | 2021-0037932 | A1 | 11 February 2021 |
| | | | | US | 2022-0256984 | A1 | 18 August 2022 |
| | | | | WO | 2017-135550 | A1 | 10 August 2017 |
| KR | 10-2018-0056714 | A | 29 May 2018 | BR | 112018005820 | A2 | 16 October 2018 |
| | | | | CN | 108024741 | A | 11 May 2018 |
| | | | | EP | 3352659 | A1 | 01 August 2018 |
| | | | | JP | 2018-531669 | A | 01 November 2018 |
| | | | | US | 10667705 | B2 | 02 June 2020 |
| | | | | US | 2017-0079534 | A1 | 23 March 2017 |
| | | | | WO | 2017-052821 | A1 | 30 March 2017 |

Form PCT/ISA/210 (patent family annex) (July 2022)